# EUROPEAN PATENT SPECIFICATION

(11) **EP 0 998 461 B1**
(45) Date of publication and mention of the grant of the patent: **14.11.2007**
(21) Application number: 98936572.1
(22) Date of filing: 22.07.1998
(51) Int. Cl.: C07D 235/02

(54) **PROCESS FOR THE PREPARATION OF IMIDAZOLONES**
VERFAHREN ZUR HERSTELLUNG VON IMIDAZOLONEN
ELABORATION D'IMIDAZOLONES

(30) Priority: 25.07.1997 HU 9701296
(43) Date of publication of application: 10.05.2000
(73) Proprietor: Sanofi-Aventis, 75013 Paris (FR)
(72) Inventor: HUSZAR, Csaba, H-1124 Budapest (HU); KIS-TAMAS, Attila, H-1035 Budapest (HU); NEMETH, Attila, H-2131 Göd (HU); GAJARY, Antal, H-1024 Budapest (HU); PALI, Lajosné, H-1043 Budapest (HU)
(74) Representative: Beetz & Partner
(86) International application number: PCT/HU1998/000065
(87) International publication number: WO 1999/005118

(56) References cited:
- US-A- 5 270 317
- C. A. BERNHART ET AL.: "A New Series of Imidazolones: Highly Specific and potent Nonpeptide AT1 Angiotensin II Receptor Antagonists" J. MED. CHEM., vol. 36, no. 22, 1993, pages 3371-3380, XP002064391
- U. SCHOELLKOPF ET AL.: "Asymmetrische Synthese von alpha-Methylphenylalanin und seinen Analoga durch Alkylieren von 1-chiral substituiertem 4-Methyl-2-imidazolon-5-on" LIEBIGS ANN. CHEM., no. 3, 1981, pages 439-458, XP002077703
- J. BRUNKEN, G. BACH: "Synthesen in der Imidazolon-Reihe" CHEM. BER., vol. 89, no. 6, 1956, pages 1363-1373, XP002077704
- P. S. KOKHLOV ET AL.: "Synthesis of phosphorylated 2-imidazolin-5-ones" J. GEN. CHEM. USSR, vol. 54, no. 12, 1984, pages 2414-2415, XP002077705
- E. GALVEZ ET AL.: "Structural study of tropane-3-spiro-4'-imidazol-5'-one" J. MOL. STRUCT., vol. 75, no. 2, 1981, pages 241-254, XP002077706
- E. SCHIPPER, A. R. DAY: "Studies in Imidazoles. II. Imidazo[b]pyrazines" J. AMER. CHEM. SOC., vol. 74, 1952, pages 350-353, XP002077707

## Description

The present invention relates to a new process for the preparation of compounds of the general formula I 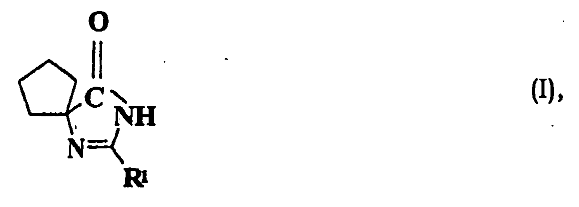 wherein R¹ is hydrogen or C₁-C₆-alkyl,
and their salts,
and to intermediate compounds therefor of the general formula (IV) 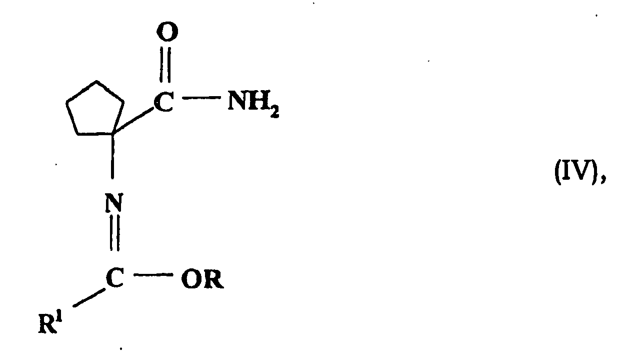 wherein R is C₁-C₄-alkyl, and R¹ is as defined above.

Compounds of general formula I are intermediates of compounds with angiotensin-II antagonist activity which are described in the Hungarian Patent No. 211839 and in the equivalent patent US 5 270 317.

US 5 270 317 generally discloses the reaction of α-aminoamides with alkyl orthoesters under acidic conditions leading to the corresponding cyclic 1,3-diaza-4-oxocycloalk-1-enes, however, without giving an example for the synthesis of compounds of the above formula I.

For a person of ordinary skil, based on the knowledge of the prior art, this reaction route, where an acidic medium is applied, does not seem to be feasible, since it has been known that orthoesters undergo decomposition by the effect of acids (Houben-Weyl, Band VI/3, S.315 (1965)). It is well-known, too, that the lone-pair of electrons of primary amines is not available for further reactions in an acidic medium, as it takes part in the salt formation. It is also known that in an acidic medium, primary amines and orthoesters also give rise to the formation of N-aryl-N-alkylformamides, from which the compounds of general formula I may not be formed (J. Am. Chem. Soc. 78, p. 4778 (1956)).

In Chem. Ber. 89, 1363 (1956), the above reaction as disclosed in US 5 270 317 is used for the preparation of 1,3-disubstituted imidazolinones under neutral reaction conditions.

Similar reactions which are performed without using acidic catalysts are disclosed in J. Gen. Chem. USSR 54, 2414 (1984).

In the knowledge of the above prior art it is the problem underlying the present invention to provide a process for the preparation of compounds of the general formula I which proceeds in good yield and results in pure products, and to provide a suitable intermediate product.

The above problem is solved according to the independent claims. The dependent claims relate to preferred embodiments of the invention.

The process of the present invention for the preparation of compounds of the general formula I 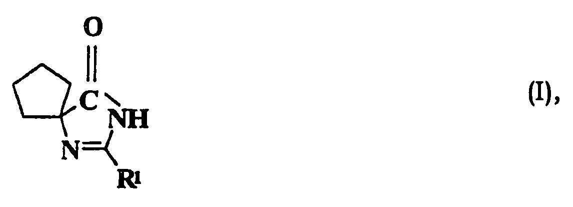 wherein R¹ is hydrogen or C₁-C₆-alkyl,
and their salts
is characterized by
reaction of a compound of formula II 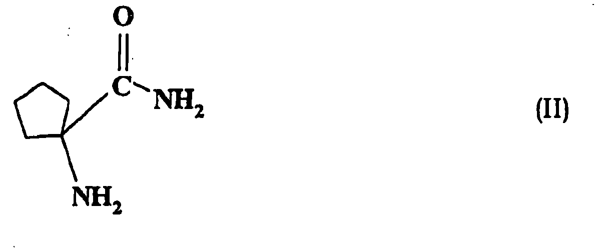 with, a compound of the general formula III, 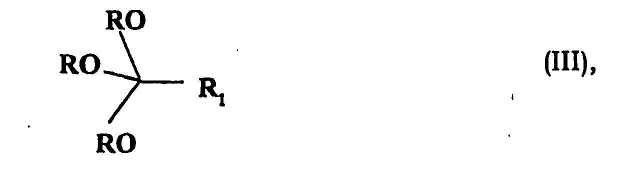 wherein
R is C₁-C₄-alkyl
   and
R¹ is as in formula I,
   by heating a mixture of compounds of formulae II and III of neutral pH at the reflux temperature of the mixture
   to obtain a compound of the general formula IV, 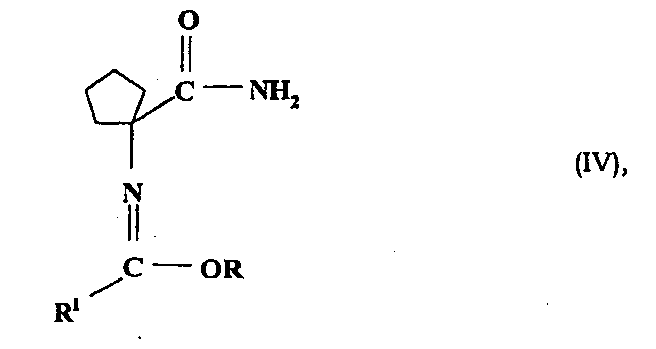 wherein R and R¹ are as defined above,
   and
   - cyclization of the compound of formula IV to obtain the compound of formula I
      by further elevating the temperature of the neutral reaction mixture,
      and optionally
   - transformation of the compound of the general formula I into a salt,
   - or liberation of the compound of the general formula I from a salt thereof.

According to a preferred embodiment, the process of the invention is characterized in that the reaction of the compound of formula II with a compound of formula III, wherein R is methyl and R¹ is n-butyl, is carried out at 70 to 80 °C under reflux conditions.

In accordance with a further preferred embodiment, the process according to the invention is characterized in that the resulting compound of the general formula I is transformed in the reaction mixture into its hydrochloride salt and isolated in the form of this salt.

The invention further relates to intermediate compounds of the general formula IV, 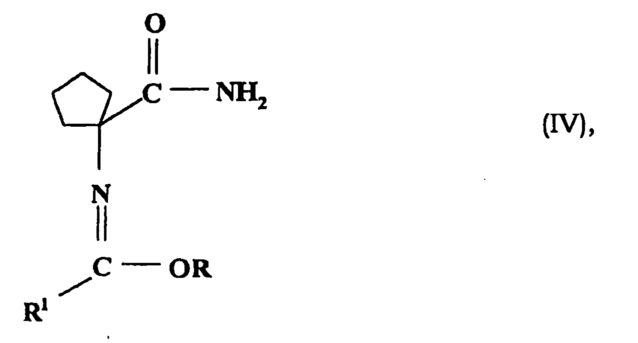 wherein
R is C₁-C₄-alkyl,
   and
R¹ is hydrogen or C₁-C₆-alkyl.

Preferred compounds of formula IV are N-(1-Carboxamidocyclopentyl-1)-formimino ethyl ether and N-(1-Carboxamidocyclopentyl-1)-pentanimino methyl ether.

The compounds of the general formula I are obtained in very pure condition, in almost theoretical yields, even with applying just equivalent amounts of reactants.

The new compounds of the general formula IV are useful intermediates in the process described above.

According to a preferred embodiment of the invention, the compound of formula II is mixed with a compound of the general formula III, the reaction is carried out at the boiling point of the mixture, the resulting compound of the general formula IV, optionally without isolation, is then cyclized by enhancing the temperature of the reaction mixture to 110-160 °C, in neutral reaction medium, under conditions of distillation or vacuum distillation. When the reaction mixture containing the resulting compounds of general formula I is acidified, the compounds of formula I are obtained in the form of their acid addition salts.

The compound of formula II can be synthesized as described in the above two patents in Examples 2a and 2b, whereas the compounds of general formula III can be prepared according to J. Am. Chem. Soc. 68, p. 1923 (1946).

Further details of the process of the invention are illustrated by the following examples, without limiting the claims to the content of examples.

### Example 1

2-Butyl-1,3-diaza-spiro[4,4]non-1-en-4-one-monohydrochloride 20 g (0,156 mol) of 1-aminocyclopentane-1-carboxamide and 31 g (0,19 mol) of trimethyl orthovalerate are kept under reflux at 70-80 °C for 1 h. The condenser is then changed to a "No hold up" condenser, while heating and stirring are continued to distill off volatile components. The reaction is completed in vacuo. The residue is then dissolved in 150 ml of acetone, the pH is adjusted to 1-2, and after cooling the resulting suspension, the product is filtered off. 31 g of the title compound are obtained, yield 86,4 %.
IR: 3600-2200: vibr, NH; 1779 : γ C = O; 1642 γ C, 1517 : δ NH (IRFT Perkin Elmer)
1H-NMR: 0.9 ppm T(CH₃), 1.34 ppm S(CH₂), 1.73 ppm Q (CH₂), 1.78-2.01 ppm M cyclopentane (CH₂); 2.78 ppm T (CH₂); 9-15 ppm (NH, N)
MS: 194, 179, 166, 165, 152, 124, 84, 83, 54, 41
TLC: eluent chloroform: methanol = 6:1, TLC plate Kieselgel GF254.
Detection: I₂ vapors Rf = 0.64.

### Example 2

1,3-Diazaspiro[4,4]non- 1-en-4-one monohydrochloride 20 g (0,156 mol) of 1-aminocyclopentane-1-carboxamide and 28 g (0,19 mol) of trimethyl orthoformate are kept under reflux for 1 h. The condenser is then changed to a "No hold up" condenser, and the temperature is elevated to 140 °C. The residue is then dissolved in 150 ml of acetone, the pH is adjusted to 1-2 with concentrated hydrochloric acid, and after cooling the resulting suspension, the product is filtered off. 25 g of the title compound are obtained; yield 92.2%.

| | |
|---|---|
| Mp: 219-221 °C (decomp). | |
| IR: | 1663 cm-¹ C = N |
| | 1739 cm-¹ C = O |
| | 3197 cm-¹ H(NH) |
| MH⁺ : | 139 |

### Example 3

N-(1-Carboxamidocyclopentyl-1)-formimino ethyl ether 12,8 g (0,1 mol) 1-amino-1-carboxamidocyclopentane and 15 g (0.101 mol) of triethyl orthoformate were heated at 80 °C for 1 h. Volatile materials formed in the reaction were distilled off in fine vacuum. The residual 18 g (97.8 %) of oily material were identified by IR and MS as the title compound.

| | |
|---|---|
| IR: | 1650 cm⁻¹ : CO(CONH) |
| | 1625 cm⁻¹ : C = N |
| | 3350 cm⁻¹ : H(amide) |
| MH⁺ : | 185 |

### Example 4

N-(1-Carboxamidocyclopentyl-1)-pentaneimino methyl ether 12,8 g (0.1 mol) of 1-amino-1-carboxamidocyclopentane and 16.2 g (0.1 mol) of trimethyl orthovalerate were stirred at 80 °C for 1 h. Volatile materials formed in the reaction were distilled off in fine vacuum. The residual 22,6 g (99 %) of oily material were identified by IR and MS as the title compound.

| | |
|---|---|
| IR: | 1650 cm⁻¹: CO(CONH) |
| | 1630 cm⁻¹: C = N |
| | 3297 cm⁻¹: H(amide) |
| MH⁺: | 227 |

## Claims

1. Process for the preparation of compounds of the general formula I 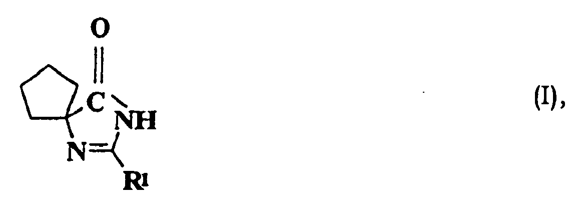 wherein R¹ is hydrogen or C₁-C₆-alkyl,
and their salts,
**characterized by**
- reaction of a compound of formula II 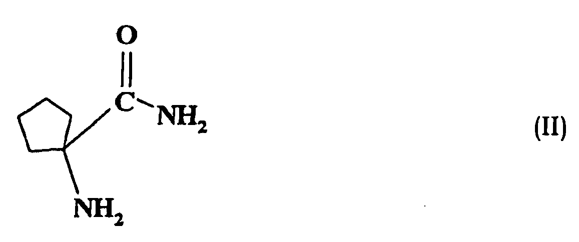 with a compound of the general formula III, 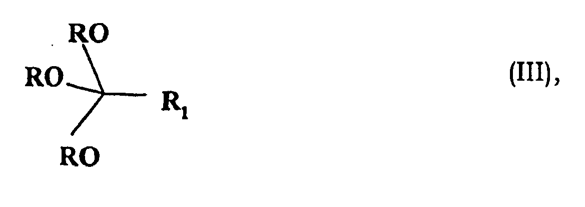
wherein
R is C₁-C₄-alkyl
and
R¹ is as in formula I,
by heating a mixture of compounds of formulae II and III of neutral pH at the boiling temperature of the mixture
to obtain a compound of the general formula IV, 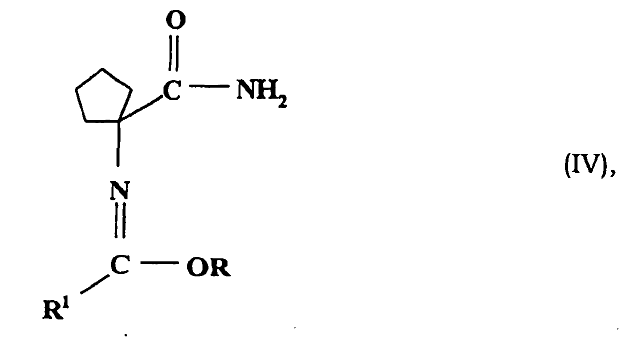 wherein R and R¹ are as defined above,
and
- cyclization of the compound of formula IV to obtain the compound of formula I
by further elevating the temperature of the neutral reaction mixture,
and optionally
- transformation of the compound of the general formula I into a salt,
- or liberation of the compound of the general formula I from a salt thereof.

2. Process according to claim 1, **characterized in that** the reaction of the compound of formula II with a compound of formula III, wherein R is methyl and R¹ is n-butyl, is carried out at 70 to 80 °C under reflux conditions.

3. Process according to claim 1 or 2, **characterized in that** the resulting compound of the general formula I is transformed in the reaction mixture into its hydrochloride salt and isolated in the form of this salt.

4. Compounds of the general formula IV, 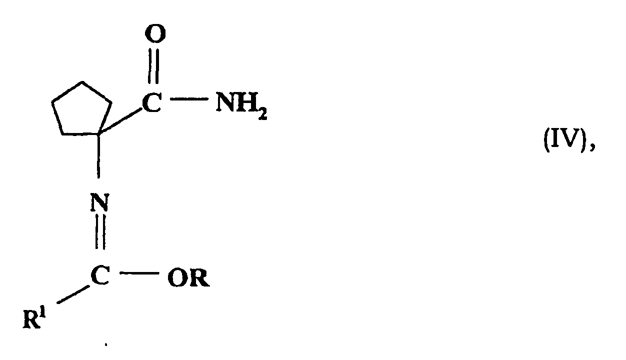 wherein
R is C₁-C₄-alkyl,
and
R¹ is hydrogen or C₁-C₆-alkyl.

5. N-(1-Carboxamidocyclopentyl-1)-formimino ethyl ether.

6. N-(1-Carboxamidocyclopentyl-1)-pentanimino methyl ether.

## Patentansprüche

1. Verfahren zur Herstellung von Verbindungen der allgemeinen Formel I, 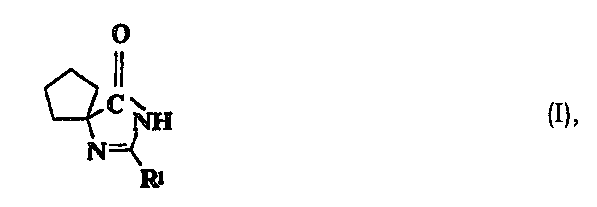 in der R¹ Wasserstoff oder C₁-C₆-Alkyl bedeutet,
sowie ihrer Salze,
**gekennzeichnet durch**
- Umsetzung einer Verbindungen der Formel II 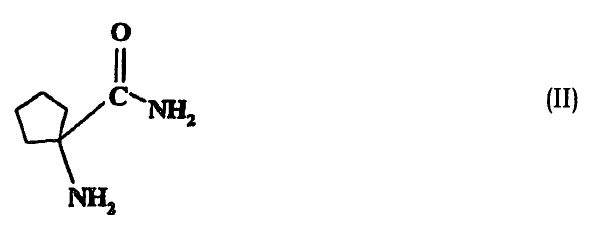 mit einer Verbindung der allgemeinen Formel III, 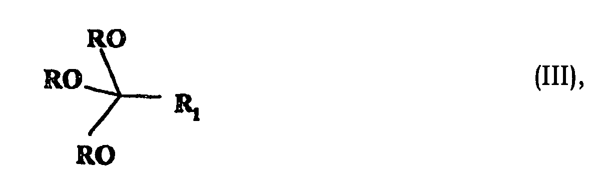
worin bedeuten:
R C₁-C₄-Alkyl
und
R¹ dasselbe wie in Formel I,
**durch** Erhitzen eines Gemischs von Verbindungen der Formeln II und III mit neutralem pH-Wert bei der Siedetemperatur des Gemisches
unter Erhalt einer Verbindung der allgemeinen Formel IV, 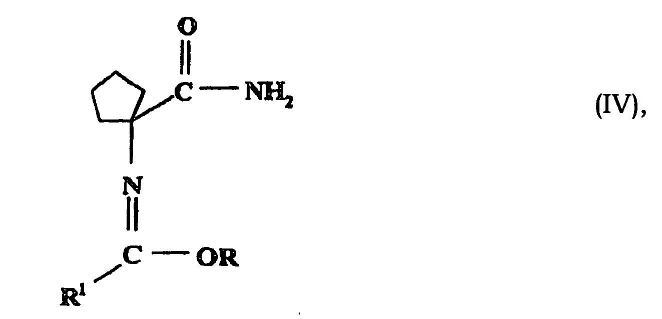 in der R und R¹ wie oben definiert sind,
und
- Cyclisierung der Verbindung der Formel IV unter Erhalt der Verbindung der Formel I **durch** weitere Erhöhung der Temperatur des neutralen Reaktionsgemischs sowie wahlweise
- Umwandlung der Verbindung der allgemeinen Formel I in ein Salz
- oder Freisetzung der Verbindung der allgemeinen Formel I aus einem Salz dieser Verbindung.

2. Verfahren nach Anspruch 1, **dadurch gekennzeichnet, dass** die Umsetzung der Verbindung der Formel II mit einer Verbindung der Formel III, worin R Methyl und R¹ n-Butyl bedeuten, bei 70 bis 80 °C unter Rückflussbedingungen durchgeführt wird.

3. Verfahren nach Anspruch 1 oder 2, **dadurch gekennzeichnet, dass** die resultierende Verbindung der allgemeinen Formel I im Reaktionsgemisch in ihr Hydrochlorid umgewandelt und in Form dieses Salzes isoliert wird.

4. Verbindungen der allgemeinen Formel IV, 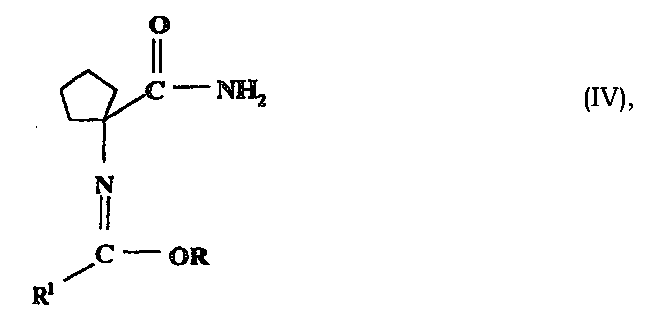 in der bedeuten:
R C₁-C₄-Alkyl
und
R¹ Wasserstoff oder C₁-C₆-Alkyl.

5. N-(1-Carboxamidocyclopentyl-1)-formininoethylether.

6. N-(1-Carboxamidocyclopentyl-1)-pentaniminomethylether.

## Revendications

1. Procédé de préparation de composés de formule générale I 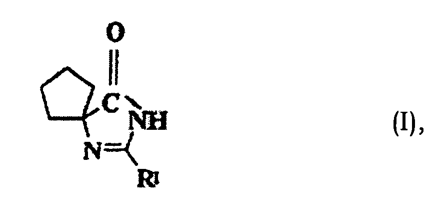 dans laquelle R¹ est un hydrogène ou un alkyle en C₁-C₆, et leurs sels,
**caractérisé par**
- la réaction d'un composé de formule II 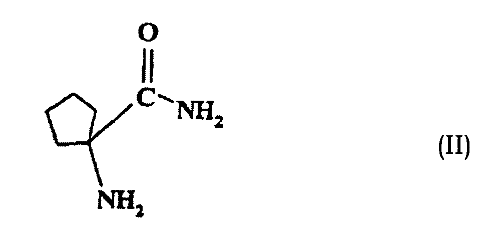 avec un composé de formule générale III 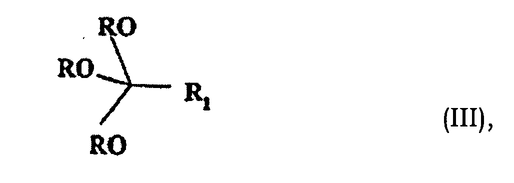
dans laquelle
R est un alkyle en C₁-C₄
et
R¹ est comme dans la formule I,
par chauffage d'un mélange des composés de formules II et III de pH neutre à la température d'ébullition du mélange pour obtenir un composé de formule générale IV, 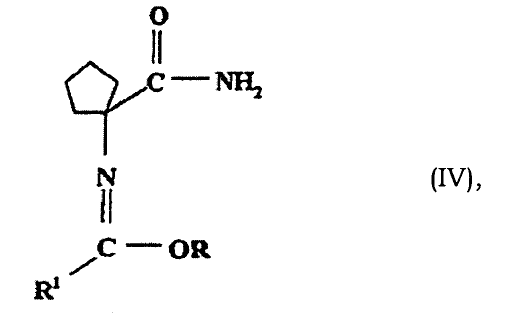 dans laquelle R et R¹ sont tels que définis ci-dessus,
et
- la cyclisation du composé de formule IV pour obtenir le composé de formule I
en élevant encore la température du mélange réactionnel neutre,
et le cas échéant
- la transformation du composé de formule générale 1 en un sel,
- ou la libération du composé de formule générale 1 d'un sel de celui-ci.

2. Procédé selon la revendication 1, **caractérisé en ce que** la réaction du composé de formule II avec un composé de formule III, dans lequel R est un méthyle et R¹ est un n-butyle, est réalisée à une température de 70 à 80°C dans des conditions de reflux.

3. Procédé selon la revendication 1 ou 2, **caractérisé en ce que** le composé résultant de la formule générale 1 est transformé dans le mélange réactionnel en son sel de chlorhydrate et isolé sous la forme de son sel.

4. Composés de formule générale IV 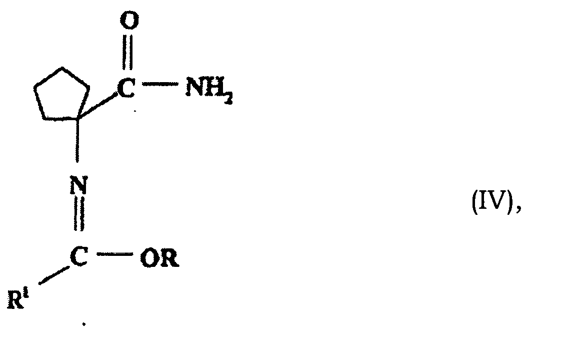 dans laquelle
R est un alkyle en C₁-C₄,
et
R¹ est un hydrogène ou un alkyle en C₁-C₆.

5. Ether N-(1-carboxamidocyclopentyl-1)-formiminoéthylique.

6. Ether N-(1-carboxamidocyclopentyl-1)-pentaniminométhylique.
